# EUROPÄISCHE PATENTSCHRIFT

(11) **EP 1 233 758 B1**
(45) Veröffentlichungstag und Bekanntmachung des Hinweises auf die Patenterteilung: **15.10.2003**
(21) Anmeldenummer: 00979241.7
(22) Anmeldetag: 16.11.2000
(51) Int. Cl.: A61K 9/16, A61K 9/20, A61K 31/19

(54) **NATRIUMVALPROAT-GRANULAT MIT VERRINGERTER HYGROSKOPIZITÄT**
SODIUM VALPROATE GRANULATE WITH REDUCED HYGROSCOPICITY
GRANULE DE VALPROATE DE SODIUM PRESENTANT UNE HYGROSCOPICITE REDUITE

(30) Priorität: 02.12.1999 AT 203099
(43) Veröffentlichungstag der Anmeldung: 28.08.2002
(73) Patentinhaber: Gerot Pharmazeutika Gesellschaft m.b.H, 1160 Wien (AT)
(72) Erfinder: RAMER, Wolfgang, A-2345 Brunn am Gebirge (AT)
(74) Vertreter: Pawloy, Peter Michael, Dipl.-Ing.
(86) Internationale Anmeldenummer: AT0000306
(87) Internationale Veröffentlichungsnummer: WO01039747

(56) Entgegenhaltungen:
- WO-A-98/40060
- DATABASE CHEMABS [Online] CHEMICAL ABSTRACTS SERVICE, COLUMBUS, OHIO, US; HASEGAWA, AKIHIKO ET AL: "Inhibition of the moisture absorption of sodium valproate by organic acid" retrieved from STN Database accession no. 108:11135 HCA XP002171181 in der Anmeldung erwähnt & YAKUZAIGAKU (1987), 47(2), 86-92 ,
- DATABASE WPI Section Ch, Week 199139 Derwent Publications Ltd., London, GB; Class A96, AN 1991-284728 XP002171182 & JP 03 188020 A (DAINIPPON PHARM CO LTD), 16. August 1991 (1991-08-16)

## Beschreibung

Die vorliegende Erfindung betrifft ein Natriumvalproat-Granulat mit verringerter Hygroskopizität sowie ein neues Verfahren zur Herstellung eines solchen Natriumvalproat-Granulates. Das erfindungsgemäße Granulat kann zu oralen pharmazeutischen Formen, insbesondere schnell-freisetzenden Tabletten aber auch Tabletten mit retardierter Freisetzung verarbeitet werden.

Valproinsäure (VPA) und seine Salze Natrium Valproat (NaVPA), Natrium Divalproex (NaHVPA), Magnesium Valproat (MgVPA) und Calcium Valproat (CaVPA) werden erfolgreich in der Behandlung epileptischer Anfälle eingesetzt. VPA wird in-Gesamttagesdosen von 500 - 3000 mg verabreicht, wobei die Einzelgaben 1000 mg nicht überschreiten sollen.

VPA und ihre Alkali- und Erdalkalisalze können zu lokalen Irridationen der Magenschleimhäute führen. Moderne, schnell freisetzende orale Darreichungsformen (immediate release) sollen daher mit einem magensaftresistenten Film überzogen sein, der eine VPA Freisetzung im Magen verhindert.

NaVPA ist eine hygroskopische, schlecht kristallisierende Substanz, die nur mit überdurchschnittlich hohem technologischen Aufwand zu Tabletten verarbeitet werden kann. Granulate können durch mehrfaches Kompaktieren oder durch nicht-wässriges Feuchtgranulieren hergestellt werden. Die meisten Verfahren erzielen aber nur Granulate mit einem Wirkstoffanteil von 40 %, die resultierenden Tabletten sind daher entsprechend groß und können vom Patienten oft nur mit Schwierigkeiten eingenommen werden.

Diese Granulate können überdies nur unter reduzierter Feuchte (kleiner 30 % r.F.) verpresst werden. Das Absenken der Raumfeuchte in einem Produktionsraum ist nur mit hohem technischen Aufwand und Kosten zu bewerkstelligen. Die hygroskopischen Tablettenkerne müssen vor dem Aufbringen des magensaftresistenten Films mit einem isolierenden Film abgedeckt werden. Die fertigen Filmtabletten müssen überdies mit feuchtigkeitsausschließenden Packmaterial konfektioniert werden (mit Aluminium/Aluminium Blister oder Röhrchen mit Trocknungsmittel). Nimmt eine konventionelle NaVPA Tablette Wasser auf, so wirkt das hydrolysierende NaVPA aus dem Kern der Filmtablette basisch und löst den magensaftresistenten Film (stabil bei pH-Werten unter 5, quellbar oder löslich bei höheren pH-Werten) von innen her auf.

Auf Grund der oben aufgezählten Probleme bei der Herstellung von NaVPA-Tabletten wurden alternative Wege zu akzeptablen, oralen Darreichungsformen gesucht:

VPA ist eine nicht-hygroskopische Flüssigkeit, die ohne Zuschlag anderer Hilfstoffe in Weichgelatine-Kapseln verfüllt werden kann. VPA Weichgelatine-Kapseln können auch magensaftresistent überzogen werden, es gibt jedoch keine akzeptablen Verfahren zur Herstellung retardierter Formen in Weichgelatine-Kapseln. Auch wenn keine zusätzlichen Hilfstoffe zur Formulierung des Kapselinhaltes verwendet werden, ergeben sich auf Grund der Dichte von VPA (0,904 g/ml) relative große Formen. Überdies können Weichgelatine-Kapseln nicht zur Dosisanpassung geteilt werden.

US-4,988,731-A, US-5,212,326-A und US-5, 019,398-A betreffen eine dimere oder oligomere Koordinationsverbindung von NaVPA und VPA, im Folgenden NaHVPA genannt. NaHVPA wird durch Auskristallisieren eines molaren 1:1 Gemisch von NaVPA und VPA aus Aceton oder Acrylnitril gewonnen. NaHVPA ist weniger hygroskopisch als NaVPA und kann zu NaHVPA Tabletten verarbeitet werden. NaHVPA wird als anticonvulsiver Wirkstoff mit eigenem INN-Namen (Divalproex Sodium) beschrieben.

In einem in US-5,017,613-A beschriebenen Verfahren wird eine Mischung von Hilfsstoffen und NaVPA (2 Teile) mit VPA (1 Teil) granuliert. Mit diesem Verfahren können sowohl schnell-freisetzende als auch retardierte Formen hergestellt werden. Die fertigen Tabletten enthalten eine deklarierte Mischung aus NaVPA und VPA.

Gemäß US-5,049,586-A werden 20 Gewichts-% Magnesiumoxid mit 60 Gewichts-% VPA granuliert und ergeben ein nicht-hygroskopisches Granulat. Im Zuge der Granulation entsteht das nicht-hygroskopische MgVPA, das auch in US-5,180,850-A beschrieben wird. Da im Verlauf einer VPA-Therapie Magnesiummangel auftreten kann, werden für MgVPA klinische Vorteile im Vergleich zu NaVPA oder VPA beschrieben.

Andere Salze (z.B. mit Calcium gemäß US-4,895,873-A oder mit Aminosäuren gemäß US 4,699,927-A) oder Derivate der VPA (Glycerylvalproat gemäß US-4,654,370-A oder Valpromid gemäß BE-854487-A) haben nur lokale Bedeutung erlangt.

In einer von Hasegawa et al., Yakuzaigaku 1987, 47(2), 86-92 beschriebenen Studie werden verschiedene organische Säuren und NaVPA in Ethanol gelöst und der Rückstand nach Abdampfen des Alkohols als Komplex mit verminderter Wasseraufnahme im Vergleich zu unbehandelten NaVPA beschrieben.

Retardformen, die NaVPA als Wirksubstanz verwenden, werden auch in anderen Patentschriften beschrieben (z.B. US-5,589,191-A und US-4,913,906-A).

Die WO 98/40060 A1 betrifft nicht-zerfließende Formulierungen, enthaltend oder bestehend aus Natriumvalproat und Cyclodextrin. Der Gehalt an Cyclodextrin soll dabei zur Bildung einer Zusammensetzung von Natriumvalproat mit Cyclodextrin in einem molaren Verhältnis von 1 : 0,01 bis 1 : 0,09 beitragen, welche Zusammensetzung das Zerfließen von Natriumvalproat vermindern soll. Gemäß Seite 3, letzter Absatz, wird zur Herstellung einer Zusammensetzung mit verzögerter Freisetzung unter anderen auch die Zugabe pharmazeutisch annehmbarer organischer Säuren, wie Zitronensäure, Weinsäure, Bernsteinsäure oder Salzen dieser Säure, vorgeschlagen. Auch Beispiel 9, Seite 5, betrifft eine derartige Zusammensetzung, gemäß diesem Beispiel 9 werden 3 kg Natriumvalproat mit 1 kg β-Cyclodextrin, 2 kg Metolose 90 und 1,15 kg wasserfreie Zitronensäure sowie 0,4 kg Kollidon in einem Fließbettgranulator granuliert. Die verminderte Hygroskopizität derartiger Formulierungen ist gemäß der Offenbarung der WO 98/40060 A1 ausschließlich auf den Gehalt an Cyclodextrin zurückzuführen.

Die WO 96/23491 A1 betrifft nicht-hygroskopische Verbindungen von Natriumvalproat und Valproinsäure, wobei die gewonnene Verbindung, Divalproex-Sodium, ohne Verwendung organischer Lösungsmittel hergestellt wird. Die Verwendung irgendwelcher pharmazeutisch annehmbarer organischer Säuren als Stabilisatoren ist diesem Dokument nicht zu entnehmen.

Die US 5 212 326 A wiederum betrifft Salze von Valproinsäure, nämlich gewisse Diethyl- oder Dipropylessigsäure-Salze von Natriumvalproat.

Die WO 94/27587 A2 offenbart bestimmte Dosierungsformen zur Verabreichung von Valproinsäure oder einem Valproinsäure-Derivat, wobei gemäß Seite 12 gewisse organische Säuren, wie Ölsäure, Caprylsäure und Stearinsäure, als Schmiermittel verwendet werden. Gemäß Seite 22, Zeilen 9 bis 15, wird das Schmiermittel (hier Magnesiumstearat) nach Abschluss der Granulierung einfach unter das fertige Granulat gemischt.

Die JP 61186313 A offenbart ebenfalls nicht-zerfließende Formulierungen, enthaltend Natriumvalproat, eine Zusammensetzung mit verbessertem Wasserhaltevermögen, sowie Wachse, höhere Fettsäuren und dgl..

Die vorliegende Erfindung stellt sich nun die Aufgabe, NaVPA-Granulate mit reduzierter Hygroskopizität (Wasserdampfempfindlichkeit) bereitzustellen.

Diese Aufgabe wird erfindungsgemäß durch ein Granulat gelöst, welches das Natriumsalz von Valproinsäure zusammen mit 5 bis 30 %-Masse im Verhältnis zum Natriumsalz von Valproinsäure an Stabilisator enthält und der Stabilisator ausgewählt ist aus der Gruppe bestehend aus Citronensäure und seine Hydrate, insbesondere das Monohydrat, Äpfelsäure und Weinsäure. Aus den erfindungsgemäßen NaVPA-Granulaten können schnell-freisetzende Tabletten aber auch Tabletten mit retardierter Freisetzung hergestellt werden. Zur Verwendung als Stabilisator sind organische Säuren geeignet, die auch als Genuss (Lebensmittel) säuren Verwendung finden, wie z.B. Citronensäure und seine Hydrate, insbesondere das Monohydrat, Äpfelsäure, Weinsäure und dgl.

Die zur Stabilisierung verwendeten organischen Säuren werden in einem Ausmaß von 5 bis 35 %-Masse eingesetzt. Die organischen Säuren werden als wässrige Lösung oder in der wässrigen Lösung des Binders aufgelöst und bei der Granulierung innig mit dem vorgelegten NaVPA gemischt.

Zusätzliche, in der Tablettierung übliche Hilfstoffe wie Füllstoffe, Sprengmittel, Gleitmittel werden dem Granulat vor und/oder nach der Feuchtgranulation zugegeben.

Auf Grund der reduzierten Feuchtigkeitsempfindlichkeit von aus dem Granulat erhaltbarer Tablettenkerne können sowohl dekorative als auch funktionelle Filme (magensaftresistente Filme) aus wässrigen Suspensionen ohne die Verwendung einer isolierenden Abdeckschicht aufgebracht werden.

Weiters betrifft die vorliegende Erfindung die Herstellung des erfindungsgemäßen NaVPA-Granulats, welches Herstellungsverfahren die folgenden Verfahrensschritte umfasst:
- Gegebenenfalls Vormischen des Natriumsalzes von Valproinsäure mit Hilfsstoffen wie z.B. Füllstoffen oder Retardierungsmitteln, wie Polymeren, zur Bildung einer inneren Phase des Granulats,
- Lösen des Stabilisators in der Granulationsflüssigkeit,
- Feuchtgranulation von NaVPA bzw. der Vormischung,
- Trocknung,
- Trockengranulation und
- gegebenenfalls Untermischen von Hilfstoffen zur Bildung einer äußeren Phase.

Die Erfindung wird durch folgende Beispiele näher "erläutert, ohne hierauf eingeschränkt zu sein.

### Beispiel 1: Schnell-freisetzende NaVPA Filmtabletten mit Citronen-säure (500 mg VPA)

435 g Citronensäure Monohydrat werden in 350 ml demineralisiertem Wasser gelöst. 100 g Polyvinylpyrrolidon 25, 2881,5 g NaVPA, 160 g amorphe Kieselsäure und 200 g mikrokristalline Cellulose werden gemischt und mit der Citronensäurelösung feuchtgranuliert. Nach dem Trocken auf Horden oder in der Wirbelschicht. wird das Granulat durch ein 1 mm Sieb gebrochen, mit der äußeren Phase (enthaltend 120 g Natriumcarboxymethylcellulose, 30 g Magnesiumstearat, 75 g Talkum und 200 g amorphe Kieselsäure) endgemischt und zu Tablettenkernen verpresst (Tablettengewicht 840,3 mg).

Eine wässrige Suspension aus 525 g wässriger Acrylharzsuspension (Methacrylic Acid Copolymer, Type C, USP XXI, Eudragit L30D), 6 g Polyvinylpyrrolidon 25, 15,75 g Dibutylphthalat, 3 g Polyethylenglykol 6000, 37 g Talkum und 3 g Titandioxid wird in einer geeigneten Einrichtung (Dragierkessel, Filmcoatinganlage) auf die Tablettenkerne aufgebracht, so daß sich ein Auftrag von 4-5 mg Eudragit L30D Trockensubstanz ergibt.

Gewicht der Filmtablette: 885 mg

Ausbeute: ca. 5000 Filmtabletten

### Zerfallszeit gemäß Eu. Pharm.:

| | |
|---|---|
| in verdünnter Salzsäure pH 1 | > 2 Stunden |
| in Phosphatpuffer pH 6,8 | < 20 Minuten |

### Wasseraufnahme:

Die unverpackten Tabletten werden bei 25°C und 60 % relativer Luftfeuchte gelagert. In bestimmten zeitlichen Abständen wird durch Wägung die Wasseraufnahme festgestellt (in Klammern zum Vergleich DEPAKOTE^{*} delayed release 500 mg Tabletten von Abbott enthaltend NaHVPA):

| | erfindungsgemäß | DEPAKOTE® |
|---|---|---|
| in 4 Stunden | 0,16 % | (0,23 %) |
| in 24 Stunden | 0,45 % | (0,88 %) |
| in 120 Stunden | 1,41 % | (2,49 %) |

### In-vitro Freisetzung:

In einer der USP XXI, System 2 (Paddle) entsprechenden Einrichtung werden aus dem erfindungsgemäßen Granulat hergestellte Tabletten in 1000 ml Phosphatpuffer, der 1 % Dodecylsulfat Natrium bei pH 6,8 enthält, bei 37°C mit 100 Upm gerührt. Die in Lösung gegangene Menge NaVPA wurde mittels einer gaschromatographischen Methode bestimmt:
in 30 Minuten: 91,9 ± 2,5 %

### Beispiel 2: Schnell-freisetzende NaVPA Filmtabletten mit Weinsäure (500 mg NaVPA)

175 g Weinsäure werden in 150 ml demineralisiertem Wasser gelöst. 1037 g NaVPA, 36 g Polyvinylpyrrolidon 25, 58 g amorphe Kieselsäure und 72 g mikrokristalline Zellulose werden gemischt und mit der Weinsäurelösung feuchtgranuliert. Nach dem Trocken auf Horden oder in der Wirbelschicht wird das Granulat durch ein 1 mm Sieb gebrochen und mit der äußeren Phase (enthaltend 43 g Natriumcarboxymethylstärke, 11 g Magnesiumstearat, 27 g Talkum und 72 g amorphe Kieselsäure) endgemischt und zu Tablettenkernen verpreßt (Tablettengewicht 822 mg).

Filmcoaten wie unter Beispiel 1.

### Zerfallszeit gemäß Eu. Pharm.:

| | |
|---|---|
| in verdünnter Salzsäure pH 1 | > 2 Stunden |
| in Phosphatpuffer pH 6,8 | < 20 Minuten |

### Wasseraufnahme:

Die unverpackten Tabletten werden bei 25°C und 60 % relativer Luftfeuchte gelagert. In bestimmten zeitlichen Abständen wird durch Wägung die Wasseraufnahme festgestellt (in Klammern zum Vergleich DEPAKOTE® delayed release 500 mg Tabletten von Abbott enthaltend NaHVPA):

| | erfindungsgemäß | DEPAKOTE® |
|---|---|---|
| in 4 Stunden | 0,18 % | (0,23 %) |
| in 24 Stunden | 0,65 % | (0,88 %) |
| in 120 Stunden | 1,90 % | (2,49 %) |

### In-vitro Freisetzung:

In einer der USP XXI, System 2 (Paddle) entsprechenden Einrichtung werden aus dem erfindungsgemäßen Granulat hergestellte Tabletten in 1000 ml Phosphatpuffer, der 1 % Dodecylsulfat Natrium bei pH 6,8 enthält, bei 37°C mit 100 Upm gerührt. Die in Lösung gegangene Menge NaVPA wurde mittels einer gaschromatographischen Methode bestimmt:
in 30 Minuten 96,2 ± 2,9 %

### Beispiel 3: NaVPA retard Filmtabletten mit Citronensäure (500 mg NaVPA)

5525 g Citronensäure Monohydrat werden in 9492 ml Eudragit RS30D gelöst. 42500 g NaVPA, 425 g amorphe Kieselsäure, 8500 g Ethylcellulose (100 cps) werden gemischt und mit der Citronensäurelösung feuchtgranuliert. Nach dem Trocken auf Horden oder in der Wirbelschicht (70°C, Restfeuchte 1,5 -2 %) wird das Granulat durch ein 1 mm Sieb gebrochen und mit der äußeren Phase (enthaltend 850 g Magnesiumstearat, 1148 g Talkum und 425 g amorphe Kieselsäure) endgemischt und zu Tablettenkernen verpreßt (Tablettengewicht 732 mg).

Eine wässrige Suspension aus 1275 g Eudragit L30 D, 30 g Polyvinylpyrrolidon, 38,3 g Dibutylphthalat, 12,8 g Polyethylenglykol 6000, 152,2 g Talkum und 85 g Titandioxid wird in einer geeigneten Einrichtung (Dragierkessel, Filmcoatinganlage) auf die Tablettenkerne aufgebracht, sodass sich ein Auftrag von 1 mg Eudragit L30D Trockensubstanz ergibt.

### Zerfallszeit gemäß Eu. Pharm:

| | |
|---|---|
| in Wasser | > 2 Stunden |

### Wasseraufnahme:

Die unverpackten Tabletten werden bei 25°C und 60 % relativer Luftfeuchte gelagert. In bestimmten zeitlichen Abständen wird durch Wägung die Wasseraufnahme festgestellt (in Klammern: Vergleichswerte EPILIM® Chrono von Sanofi enthaltend eine Mischung von Hilfsstoffen und NaVPA (2 Teile) mit VPA (1 Teil)).

| | erfindungsgemäß | EPILIM® |
|---|---|---|
| in 4 Stunden | 0,6 % | (1,3 %) |
| in 24 Stunden | 3,3 % | (4,6 %) |

### In-vitro Freisetzung:

In einer der USP XXI, System 2 (Paddle) entsprechenden Einrichtung werden aus dem erfindungsgemäßen Granulat hergestellte Tabletten in 1000 ml Phosphatpuffer, der 1 % Dodecylsulfat Natrium bei pH 6,8 enthält, bei 37°C mit 100 Upm gerührt. Die in Lösung gegangene Menge NaVPA wurde mittels einer gaschromatographischen Methode bestimmt :

| | |
|---|---|
| nach 60 Minuten | 29,4 ± 0,8 % |
| nach 180 Minuten | 42,1 ± 1,2 % |
| nach 240 Minuten | 56,0 ± 1,8 % |

### Beispiel 4: In vivo-Bioverfügbarkeitsstudie mit Tabletten aus Beispiel 1

In einer pharmakokinetischen Pilot-Studie (Studiencode VPA/G_{P} 30-97) wurden im randomisierten Crossover-Design mit zwei Behandlungsphasen an 6 freiwilligen, männlichen Probanden die Bioverfügbarkeit bzw. das pharmakokinetische Verhalten bei einmaliger Gabe von DEPAKOTE® delayed release 500 mg (enthaltend NaHVPA entsprechend 500 mg VPA,) und einer aus dem erfindungsgemäßen Granulat hergestellten Tablette (enthaltend 500 mg NaVPA) verglichen. An den Untersuchungstagen wurden standardisierte Mahlzeiten und Flüssigkeitsmengen zu definierten Zeitpunkten eingenommen. Nach der Applikation erfolgen zu festgelegten Zeitpunkten Blutabnahmen (vor Applikation der Substanzen und 0,25, 0,5, 1, 1,5, 2, 2,5, 3, 4, 6, 8, 12, 24, 36, 48 und 60 Stunden nach Einnahme). Die Blutproben wurden in heparinisierte Röhrchen abgenommen und unmittelbar nach der Abnahme zentrifugiert. Die Plasmaproben werden bis zur Analyse tiefgekühlt aufbewahrt.

Die Analyse der Plasmaproben erfolgt mittels GC.

### Ergebnisse: (nach Dosiskorrektur entsprechend 500 mg VPA)

| Parameter | Einheiten | DEPAKOTE® | erfindungsgemäß |
|---|---|---|---|
| t_{1/2} | [h] | 16,7 | 16,7 |
| t_{lag} | [h] | 2,2 | 1,8 |
| tₘₐₓ | (h] | 3,7 | 3,2 |
| Cₘₐₓ | [mg VPA/l] | 52,6 | 53,9 |
| AUC₆₀ | [mg VPA/l*h] | 832 | 816 |
| AUC_{0-∞} | [mg VPA/l*h] | 920 | 895 |
| MRT | [h] | 17,7 | 17,1 |

### Beispiel 5: In vivo-Bioverfügbarkeitsstudie mit Tabletten aus Beispiel 3

in einer Bioäquivalenzstudie (Studiencode VPA/GP 36-99 - randomisiertes Crossover-Design in 12 freiwilligen, männlichen Probanden) wurde das in vivo-Absorptionsverhalten bei Gabe von je 2 aus dem erfindungsgemäßen Granulat hergestellten Tabletten (enthaltend je 500 mg NaVPA) und ERGENYL® Chrono 500 mg von SA-NOFI (enthaltend eine Mischung von Hilfsstoffen mit NaVPA (2 Teile) und VPA (1 Teil); jede Tablette entsprechend 500 mg NaVPA) nach nüchterner Einnahme untersucht. Nach der Applikation erfolgen zu festgelegten Zeitpunkten Blutabnahmen (vor Applikation der Prüfpräparate und 0,5, 1, 1,5, 2, 2,5, 3, 4, 6, 8, 10, 12, 15, 24, 36, 48, 60 und 72 Stunden nach Einnahme). Probenbehandlung und Analyse wie unter Beispiel 4.

### Ergebnisse:

| Parameter | Einheiten | ERGENYL® | erfindungsgemäß |
|---|---|---|---|
| t_{1/2} | [h] | 16,8 | 16,7 |
| tₘₐₓ | [h] | 9,0 | 10,0 |
| Cₘₐₓ | [mg VPA/l] | 46,6 | 45,8 |
| AUC_{0-∞} | [mg VPA/l*h] | 1529 | 1610 |
| MRT | [h] | 27,1 | 28,2 |

## Patentansprüche

1. Natriumvalproat-Granulat mit verringerter Hygroskopizität, **dadurch gekennzeichnet, dass** das Granulat das Natriumsalz von Valproinsäure zusammen mit 5 bis 30 %-Masse im Verhältnis zum Natriumsalz von Valproinsäure an Stabilisator enthält und der Stabilisator ausgewählt ist aus der Gruppe bestehend aus Citronensäure und seine Hydrate, insbesondere das Monohydrat, Äpfelsäure und Weinsäure.

2. Verfahren zur Herstellung eines Natriumvalproat-Granulats nach Anspruch 1, **dadurch gekennzeichnet, dass** das Verfahren die folgenden Verfahrensschritte umfasst:
• Gegebenenfalls Vormischen des Natriumsalzes von Valproinsäure mit Hilfsstoffen wie z.B. Füllstoffen oder Retardierungsmitteln, wie Polymeren, zur Bildung einer inneren Phase des Granulats,
• Lösen des Stabilisators in der Granulationsflüssigkeit,
• Feuchtgranulation des Natriumsalzes von Valproinsäure bzw. der Vormischung,
• Trocknung,
• Trockengranulation und
• gegebenenfalls Untermischen von Hilfstoffen zur Bildung einer äußeren Phase

## Claims

1. Granular sodium valproate material having a reduced hygroscopicity, **characterised in that** the granular material comprises the sodium salt of valproic acid together with 5 to 30% by mass of a stabilizer, relative to the sodium salt of valproic acid, said stabilizer being selected from the group consisting of citric acid and its hydrates, in particular the monohydrate, malic acid and tartaric acid.

2. A method of producing a granular sodium valproate material according to claim 1, **characterised in that** the method comprises the following method steps:
• optionally pre-mixing the sodium salt of valproic acid with auxiliary agents, such as, e.g., fillers or retarding agents, such as polymers, so as to form an inner phase of the granular material,
• dissolving the stabilizer in the granulation liquid,
• wet granulating the sodium salt of valproic acid or the premixture, respectively,
• drying,
• dry granulating and
• optionally admixing auxiliary agents so as to form an external phase.

## Revendications

1. Granulé de valproate de sodium ayant une hygroscopie réduite, **caractérisé en ce que** le granulé contient le valproate de sodium avec 5 à 30 % en masse d'un stabilisant par rapport au valproate de sodium et le stabilisant est choisi dans le groupe constitué par l'acide citrique et ses hydrates, en particulier le monohydrate, l'acide malique et l'acide tartrique.

2. Procédé de préparation d'un granulé de valproate de sodium selon la revendication 1, **caractérisé en ce que** le procédé comprend les étapes de procédé suivantes:
• éventuellement un mélange préalable du valproate de sodium avec des agents auxiliaires comme, par exemple, des charges ou des agents retardants, comme des polymères, pour la formation d'une phase interne du granulé,
• une dissolution du stabilisant dans le liquide de granulation,
• une granulation par voie humide du valproate de sodium ou du mélange préalable,
• un séchage,
• une granulation par voie sèche et
• éventuellement l'incorporation d'agents auxiliaires pour la formation d'une phase externe.
